# EUROPEAN PATENT APPLICATION

(11) **EP 4 578 834 A1**
(43) Date of publication of application: **02.07.2025**
(21) Application number: 24223013.4
(22) Date of filing: 23.12.2024
(51) Int. Cl.: C02F 1/22, G01N 33/18, C02F 1/44

(54) **METHOD OF ANALYZING MICROPLASTIC PARTICLES IN WATER SYSTEM**

(30) Priority: 28.12.2023 KR 20230195465
(71) Applicant: SK Innovation Co., Ltd., Seoul 03188 (KR)
(72) Inventor: SONG, Jae Yang, 34124 Daejeon (KR); LA, Yeon Hwa, 34124 Daejeon (KR); SIM, A Rum, 34124 Daejeon (KR); KIM, Tae Wan, 34124 Daejeon (KR); ROH, Hye Lin, 34124 Daejeon (KR)
(74) Representative: Prüfer & Partner mbB Patentanwälte · Rechtsanwälte

(57) **Abstract**

Proposed is a method of analyzing microplastic particles in a water system. The method includes providing to-be-treated water containing microplastic particles, freezing and thawing the to-be-treated water at least once to generate microplastic aggregates in the to-be-treated water, recovering the microplastic aggregates, and analyzing the recovered microplastic aggregates. According to the method, the microplastic particles are precipitated in the water system without the use of an additional coagulant, whereby the effect of coagulants on the subsequently recovered microplastic aggregates may be ruled out. In addition, the process configuration is simple because the aggregates can be separated from the supernatant without using any additional treatment process.

## Description

### BACKGROUND OF THE DISCLOSURE

### 1. Field of the Disclosure

The present disclosure relates to a method of analyzing microplastic particles in a water system.

### 2. Description of the Related Art

Globally, the use of disposable products, especially plastic disposable products such as polyethylene terephthalate (PET), has been significantly increasing. Accordingly, environmental pollution problems such as microplastic-caused water pollution are emerging. Research and development on methods of analyzing microplastic particles in a water system is also being actively conducted.

In particular, among microplastic particles in a water system, microplastic particles with sizes of several micrometers or more may be separated using a filter and analyzed using conventional analysis techniques. However, nano-sized microplastic particles that cannot be filtered by filters are not easy to separate from the water system. Therefore, research is being actively conducted on techniques to separate and analyze nano-sized microplastic particles from the water system.

Thus, there is a need for an improved method of separating microplastic particles, particularly nano-sized microplastic particles, from the environment, particularly from the water system, and of analyzing these microplastic particles in order to obtain e.g., qualitative and quantitative information. Based on the respective information obtained, a skilled person may be able to select suitable methods for removing said microplastic particles from the environment, or for otherwise making use or reuse of said microplastic particles.

### [Related Art Document]

### [Patent Document]

(Patent document 1) US 2022-0127129 A1

### SUMMARY OF THE DISCLOSURE

According to one aspect of the present disclosure, there is provided a simplified and highly accurate method of analyzing microplastic particles contained in a water system.

In one embodiment of the present disclosure, a method of analyzing microplastic particles in a water system may include: providing to-be-treated water containing microplastic particles; freezing and thawing the to-be-treated water at least once to generate microplastic aggregates in the to-be-treated water; recovering the microplastic aggregates; and analyzing the recovered microplastic aggregates, e.g., particularly regarding various characteristics, such as , but not limited to, quality and quantity, for example the size, number, and/or type of the microplastic aggregates. In a further embodiment, precipitation of the microplastic particles/aggregates thereof occurs without the use of additional coagulants. Thus, the effect of coagulants on the recovered microplastic aggregates may be ruled out.

Within the meaning of the present invention, the term "to-be-treated water" defines water that potentially comprises microplastic particles and/or other microplastic impurities, which subsequently may be analyzed.

In a further embodiment, the method may further include removing an impurity on the surface of the microplastic particles in the to-be-treated water before the freezing and thawing.

In a yet further embodiment, the removal of the impurity on the surface of the microplastic particles may be performed by any one of a strong acid or strong alkali treatment, an ultrasonic treatment, a plasma treatment, an electrochemical oxidation treatment, and an enzyme treatment, or a combination thereof.

In a yet further embodiment, the method may further include filtering the to-be-treated water before the freezing and thawing.

In a yet further embodiment, a filter used in the filtering may have a pore size of less than 25 µm.

In a yet further embodiment, the freezing and thawing of the to-be-treated water may be repeatedly performed until the number of microplastic particles in the supernatant of the to-be-treated water becomes 1 × 10¹⁰ pieces/mL or less.

In a yet further embodiment, the freezing and thawing the to-be-treated water is repeated at least twice, or 1 to 20 times, 1 to 15 times, or 5 to 10 times.

In a yet further embodiment, the freezing of the to-be-treated water may be performed at a temperature of 0°C or lower, and the thawing of the to-be-treated water may be performed at room temperature.

In a yet further embodiment, the recovering of microplastic aggregates may be performed using a capture filter with a pore size of 10 to 50 µm.

In a yet further embodiment, the analyzing of the recovered microplastic aggregates may be performed by any one of Fourier transform IR spectroscopy (FTIR), Image-FTIR, Raman spectroscopy, and pyrolysis gas chromatography/mass spectrometry, or a combination thereof.

In a yet further embodiment, the analyzing of the recovered microplastic aggregates may be performed by image-FTIR.

According to the embodiments of the present disclosure, the microplastic analysis method can be performed with high accuracy in a water system while using simplified equipment and process configuration.

In a further aspect, the present disclosure provides a method of preparing water purified from microplastic, and/or of recovering microplastic from a water system, wherein the to-be-treated water contains microplastic particles, the method comprising: providing to-be-treated water containing microplastic particles; freezing and thawing the to-be-treated water at least once to generate microplastic aggregates in the to-be-treated water; and recovering the microplastic aggregates and obtaining water purified from microplastic. In this method, analyzing the size, number, and type of the recovered microplastic aggregates may be omitted, however optionally the recovered microplastic aggregates may be analyzed where desired.

According to an embodiment, the method of preparing purified water, and/or of recovering microplastic from a water system is carried out without the use of an additional coagulant, whereby the recovered microplastic aggregates are free of coagulants.

According to a further embodiment, respectively in the method of analyzing microplastic particles in a water system and/or in the method of preparing purified water from a water system, the microplastic particles contained in the to-be-treated water has a particle size in a range of 10 nm to 10 cm, or in a range of 50 nm to 1000 nm, or in a range of 100 to 400 nm, respectively based on the largest dimension.

The methods of the present disclosure can provide multiple advantages. For example, the process configuration is simple because the aggregates formed upon applying the method of the present invention can be separated from a supernatant without using any additional treatment process. Moreover, based on the obtained result, for example it will then be possible for a skilled person to select and apply appropriate microplastic-removal methods. In particular, the method of the present disclosure can provide microplastic particle aggregates without the use of an additional coagulant, whereby the subsequently recovered microplastic aggregates are free of coagulants.

### BRIEF DESCRIPTION OF THE DRAWINGS

FIG. 1 shows a process flow diagram of a method of analyzing microplastic particles in a water system, according to one embodiment;
FIG. 2 shows an image of the turbidity degree of to-be-treated water visible to the naked eye depending on the cycle of freezing and thawing, according to a further embodiment;
FIG. 3 shows a graph illustrating change in the number of microplastic particles per unit volume of supernatant of to-be-treated water depending on the cycle of freezing and thawing, according to a yet further embodiment;
FIGs. 4, 5A to 7A, and 5B to 7B show scanning electron microscope (SEM) images of microplastic aggregates depending on the cycle of freezing and thawing, according to a yet further embodiment; and
FIGs. 8, 9A to 11A, and 9B to 11B show FTIR images of microplastic aggregates in the to-be-treated water depending on the cycle of freezing and thawing, according to a yet further embodiment.

### DESCRIPTION OF THE PREFERRED EMBODIMENTS

The purposes, advantages, and features of the present disclosure will become more apparent from the following detailed description and preferred embodiments taken in conjunction with the accompanying drawings, but the present disclosure is not necessarily limited thereto. Additionally, in describing the present disclosure, when it is determined that a detailed description of related known techniques may unnecessarily obscure the gist of the present disclosure, the detailed description will be omitted.

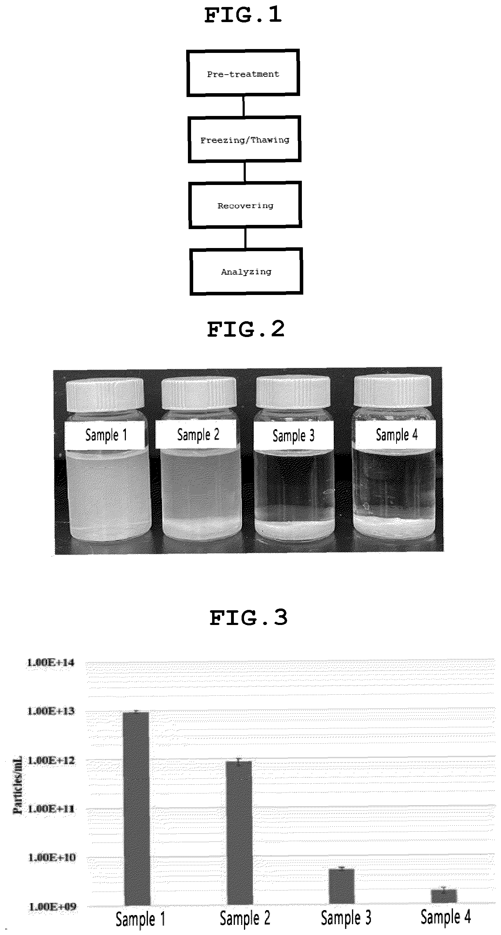

According to a method of analyzing microplastic particles in the water system of the present disclosure, the method includes: providing to-be-treated water containing microplastic particles; freezing and thawing the to-be-treated water at least once to generate microplastic aggregates in the to-be-treated water; recovering the microplastic aggregates; and analyzing the recovered microplastic aggregates. The method may be performed in the same order as the flow chart shown in FIG. 1.

The method includes providing to-be-treated water containing microplastic particles. In the present disclosure, "microplastic" refers to a plastic particle in to-be-treated water to be introduced into (subjected to) the treatment of the present disclosure. There is no limit to the type and size of the microplastic particles in the to-be-treated water as described in the steps. When indications on particle sizes are described in the present disclosure, typically the size based on the largest dimension is meant, regardless of the particle shape. For example, the microplastic particles may include not only an amorphous plastic used in disposable packs but also a crystalline plastic such as those used in beverage bottles. The microplastic particles may have a particle size in a range of a few nanometers (nm) to a few centimeters (cm). For example, microplastic particles may have a particle size in a range of 10 nanometers (nm) to 10 centimeters (cm), or in a range of 10 nanometers (nm) to 1 centimeter (cm), respectively based on the largest dimension. In one embodiment, the microplastic particles that are present in the to-be-treated water have a size of 50 nm to 1000 nm, 100 nm to 750 nm, or 100 to 400 nm, respectively based on the largest dimension. It is possible to set the particle size of the to-be-analyzed microplastic particles to a narrower range, e.g., by filtering the to-be-treated water with a filter having a predetermined pore size. Hence, by this treatment, it is possible to remove from the to-be-treated water microplastic particles that exceed a certain, predetermined size (based on the largest dimension).

The size of the particles as well as the size of the microplastic particle aggregates which form after the freezing and thawing process of the claimed method can be measured by any suitable method that is known to a skilled person. An example of such a method is Fourier transform IR spectroscopy (FTIR), Image-FTIR, Raman spectroscopy, pyrolysis gas chromatography/mass spectrometry, or a combination thereof. These methods can be used for analyzing the size of the microplastic particles, as well as the size of the microplastic particle aggregates. As disclosed elsewhere herein, it is also possible to identify the type of the microplastic particles.

In one embodiment, the method may further include removing an impurity on the surface of the microplastic particles in the to-be-treated water before the freezing and thawing. In addition to the microplastic particles that are the subject of analysis in the present disclosure, the to-be-treated water may contain an impurity such as beverages remaining when plastic was used in form of a beverage bottle. Further examples of possible impurities can be of plant origin, such as plant growth (e.g., moss or algae), but the impurities can also be of animal origin. The impurity may stick to the surface of the microplastic particles and act as noise in the subsequent analysis results for microplastic particles. Accordingly, it is preferable to remove the impurity. That is, the method further includes removing an impurity on the surface of the microplastic particles in to-be-treated water before the freezing and thawing. By doing so, it enables the recovery of pure microplastic particles after removing the impurity on the surface thereof and the use of the pure microplastic particles as an analysis subject to obtain more accurate analysis results. In a preferred embodiment, the impurity that is present on the surface of the microplastic may be removed to such an extent that upon visual inspection of a fixed number of randomly selected areas, e.g., on the surface of microplastics or in mixed form, so that essentially no impurity or no any impurity can be detected. The visual inspection may for example be carried out by applying using the scanning electron microscope (SEM) method, optionally combined with the energy dispersive X-ray spectroscopy (EDS) method.

In a further embodiment, the removal of the impurity on the surface of the microplastic particles may be performed by any one of a strong acid or strong alkali treatment, an ultrasonic treatment, a plasma treatment, an electrochemical oxidation treatment, and an enzyme treatment, or a combination thereof. The term "strong acid" may refer to an acid with a pH of 2 or lower, and the term "strong base" may refer to a base with a pH of 10 or higher. For example, the strong acid may be hydrochloric acid, and the strong base may be sodium hydroxide, without being limited thereto. The strong acid or strong alkali treatment involves adding a strong acid or strong alkali to the to-be-treated water to remove an impurity soluble in acid or alkali and adhering to the surface of the microplastic particles. The ultrasonic treatment and plasma treatment involve separating an impurity from the surface of the microplastic particles through physical methods. The electrochemical oxidation treatment involves immersing an electrode in to-be-treated water and applying power to generate free radicals when the impurity on the surface of the microplastic particles is an organic substance, and then oxidizing and removing the impurity through the oxidation reaction of the organic substance by free radicals. The enzyme treatment involves removing the impurity on the surface of the microplastic particles by adding an enzyme to the to-be-treated water, the enzyme having substrate specificity for the impurity on the surface of the microplastic particles. Two or more methods for removing the impurity on the surface of the microplastic particles as described above may be used in combination. The methods may selectively remove the impurity without damaging microplastic particles.

In a yet further embodiment, the method may further include filtering the to-be-treated water before the freezing and thawing. The analysis target of the present disclosure is a microplastic particles. Herein, the microplastic particles may be a plastic with a size of less than 25 micrometers based on the largest dimension. The method may filter the to-be-treated water using a filter with a predetermined pore size to separate larger plastic particles from the to-be-analyzed microplastic particles.

In a yet further embodiment, the filter used in the filtering may contain pores of less than 25 µm. The method of the present disclosure may include removing microplastic particles with a size of 25 µm or more based on the largest dimension from to-be-treated water through filtering using the filter having a pore with a size of less than 25 µm and analyzing microplastic particles only with a size of less than 25 µm. Specifically, the filter used in the filtering may have a pore with a size of less than 10 µm, more specifically less than 1 µm. This allows the particle size of the to-be-analyzed microplastic particles to be set to a narrower range.

The method includes freezing and thawing the to-be-treated water at least once to generate microplastic aggregates in the to-be-treated water. In the freezing and thawing, the microplastic aggregates are generated in the to-be-treated water in a thawed state after the to-be-treated water has been subjected to one or more freezing and thawing cycles. Remarkably, the microplastic particles (microplastic particle aggregates) can be precipitated in the water system without the use of an additional coagulant, whereby the effect of coagulants on the subsequently recovered microplastic aggregates may be ruled out. Regarding the generated microplastic aggregates, as the cycle of the freezing and thawing increases, the absolute number of microplastic particles in the to-be-treated water that form the microplastic aggregates increases. As the number of microplastic particles forming the microplastic aggregates increases, the size of the aggregates also increases. However, the degree of increase in aggregate size may not be significant. Referring to FIG. 2, it is visually confirmed that as the cycle of the freezing and thawing increases, the turbidity of the supernatant separated from the aggregates by precipitation decreases. From this, it is inferred that as the cycle increases, the absolute number of microplastic particles in to-be-treated water that form the microplastic aggregates increases. In a yet further embodiment, the freezing and thawing of the to-be-treated water may be specifically performed at least once, preferably at least twice, or 1 to 20 times, 1 to 15 times, and more specifically 5 to 10 times.

In a yet further embodiment, the microplastic aggregates may have a size greater than 25 µm and 10,000 µm or less based on the largest dimension. The microplastic aggregates generated during the freezing and thawing have the same size as above. The microplastic aggregates may be introduced into the analysis without any additional processing or screening. Specifically, the microplastic aggregates may have a size of 100 µm or more and 5,000 µm or less, more specifically 500 µm or more and 3,000 µm or less, based on the largest dimension.

In a yet further embodiment, the freezing and thawing of the to-be-treated water may be repeatedly performed until the number of microplastic particles in the supernatant of the to-be-treated water becomes 1×10¹⁰ pieces/mL or less. As mentioned above, as the cycle of the freezing and thawing increases, the absolute number of microplastic particles in to-be-treated water that form the microplastic aggregates increases. This means that the number of microplastic particles in the supernatant of to-be-treated water decreased. By repeating the freezing and thawing as described above until the number of microplastic particles in the supernatant becomes less than 1 × 10¹⁰/mL, the number of microplastic particles involved in aggregation increases. This may increase the analysis accuracy for the microplastic aggregates. This may also increase the recovery rate of the microplastic material, and the degree of purified water. In addition, through the repetition of the freezing and thawing, a supernatant with a greatly reduced concentration of the microplastic particles may be obtained. Therefore, the method is also further applicable to the environmentally friendly water treatment field. Specifically, the freezing and thawing of the to-be-treated water may be repeatedly performed until the number of microplastic particles in the supernatant of the to-be-treated water becomes 5×10⁹ pieces/mL or less, more specifically, 1 × 10⁹ pieces/mL or less.

In a yet further embodiment, the freezing of the to-be-treated water may be performed at a temperature of 0°C or lower, and the thawing of the to-be-treated water may be performed at room temperature. Specifically, the freezing of the to-be-treated water may be performed at a temperature of - 20°C or more and 0°C or less, or more specifically, -10°C or more and -5°C or less. When the freezing of the to-be-treated water is performed at a temperature above 0°C, the temperature may exceed the freezing point of the to-be-treated water, thereby a freezing reaction may not be carried out smoothly. When the freezing of the to-be-treated water is performed at a temperature below -20°C, aggregation may be not effective compared to the energy consumed to lower a temperature, thereby it may be energy inefficient. In addition, the thawing of the to-be-treated water specifically may be performed at a temperature of 20°C or more and 40°C or less, or more specifically, 25°C or more and 35°C or less. When the thawing of the to-be-treated water is performed at a temperature below 20°C, it may take substantial time to thaw the to-be-treated water, thereby the time required for the entire process may increase while repeating the freezing and thawing. This may lead to an increase in process costs. When the thawing of the to-be-treated water is performed at a temperature exceeding 40°C, the high-temperature effect on the aggregates may be not significant compared to the energy consumed to raise a temperature, thereby it may be energy inefficient.

The method includes recovering the microplastic aggregates. The microplastic aggregates generated in the freezing and thawing are precipitated at the bottom of the to-be-treated water. In the recovering of the microplastic aggregates, there is no limit to a method as long as the precipitated microplastic aggregates may be separated and recovered from the supernatant of the to-be-treated water.

In a yet further embodiment, the recovering of microplastic aggregates may be performed using a capture filter with a pore size of 10 to 50 µm. As described above, the microplastic aggregates in a yet further embodiment have a size greater than 25 µm based on the largest dimension. When the capture filter is used, the supernatant of the to-be-treated water and microplastic particles may be separated simply by passing the microplastic aggregates through the capture filter. Afterward, the microplastic aggregates may be analyzed without any additional processing. The recovering of the microplastic aggregates may be performed using a capture filter with a pore size of 15 to 40 µm, more specifically 20 to 30 µm, and even more specifically 10 to 25 µm.

The method includes analyzing the recovered microplastic aggregates. The analyzing of the microplastic aggregates includes qualitative as well as quantitative analysis. The quantitative analysis means the identification of the size and number of microplastic particles contained in the recovered microplastic aggregates. The qualitative analysis means the identification of the types of plastic contained in the recovered microplastic aggregates. In the analysis of the microplastic aggregates, the quantitative analysis and qualitative analysis of the microplastic aggregates may be performed simultaneously.

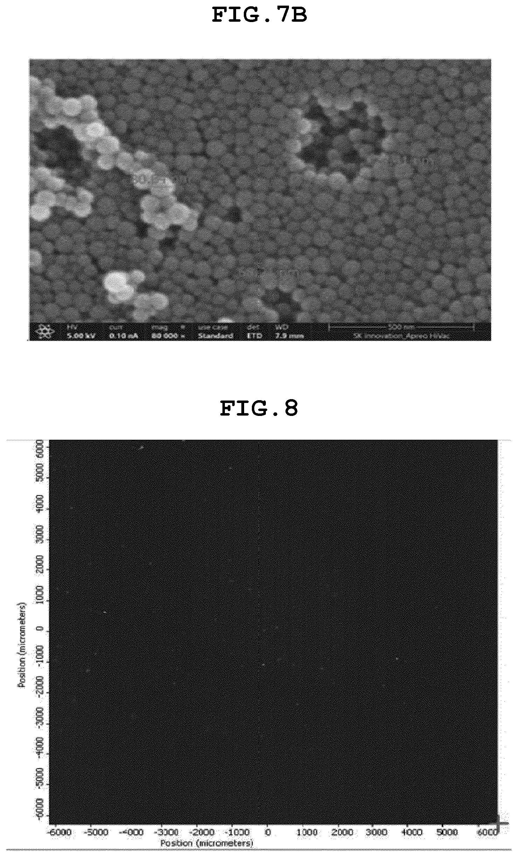

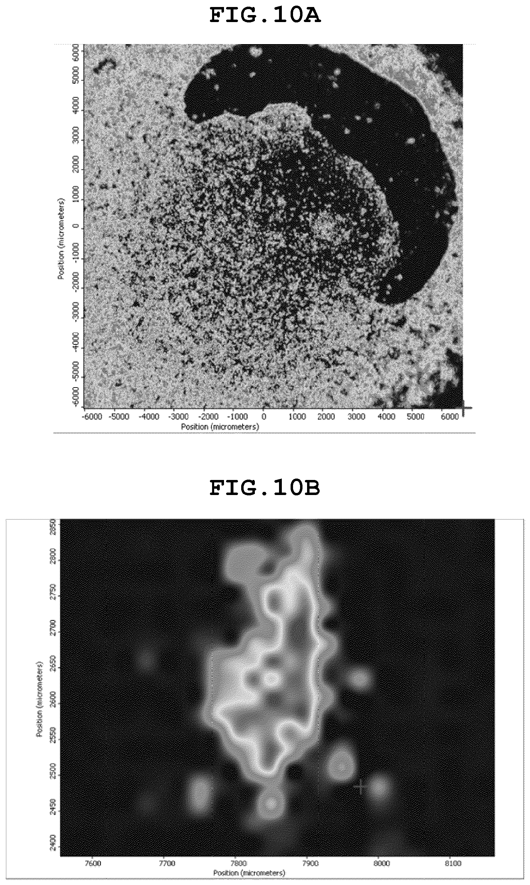

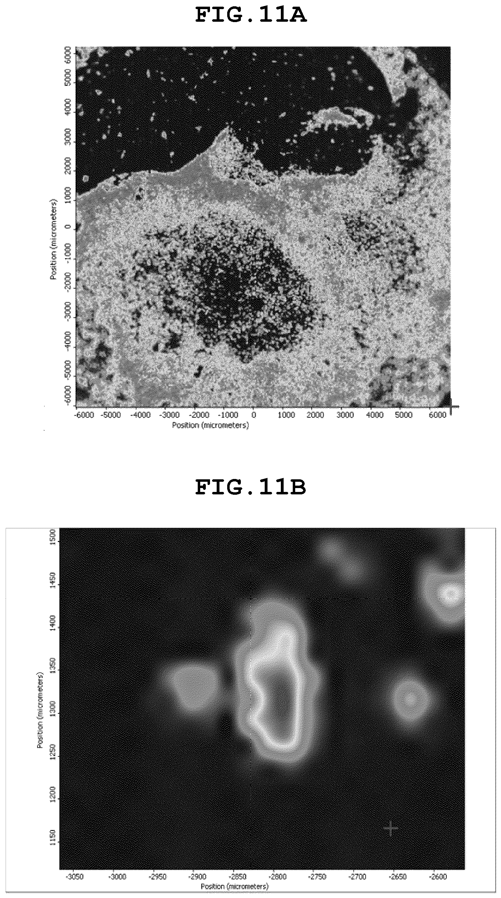

In a yet further embodiment, the analyzing of the recovered microplastic aggregates may be performed by any one of Fourier transform IR spectroscopy (FTIR), Image-FTIR, Raman spectroscopy, pyrolysis gas chromatography/mass spectrometry, or a combination thereof. The Fourier transform IR spectroscopy (FTIR) involves irradiating the microplastic aggregates with white light in the Fourier transform IR region, causing absorption depending on each material, and obtaining a unique spectrum for each material accordingly and then a spectrum for each component of the material. By this method, the type and number of microplastic particles corresponding to the peaks of each spectrum are identified. The Image-FTIR is an analyzing method that identifies the type and number of microplastic particles by imaging the FTIR spectrum as shown in FIGs. 8 to 11B. The Raman spectroscopy is an analyzing method that classifies the types of microplastic particles contained in microplastic aggregates depending on the scattered degree of incident light by irradiating a laser to microplastic aggregates. The pyrolysis gas chromatography/mass spectrometry involves breaking down microplastic aggregates through thermal decomposition and detecting peaks originating from these microplastic particles. By this method, the type of microplastic particles is identified and then information about the mass of each microplastic particle based on the sum of the areas of the peaks is obtained. Through the analyzing methods, it is possible to simultaneously analyze the qualitative and quantitative information of the microplastic particles in the water system. Considering the analysis results, appropriate microplastic removal methods may be introduced for to-be-treated water.

In a yet further embodiment, the analyzing of the recovered microplastic aggregates may be performed by image-FTIR. The image-FTIR is beneficial because analysis results are obtained even with a small amount of sample compared to the other analyzing methods mentioned above. The method is also beneficial because a more detailed analysis is provided of the aggregates, such as microplastic particle size, number, and type of plastic that makes up the microplastic aggregates.

Alternatively to the analyzing step, or optionally in addition to the analyzing step, the microplastic aggregates may be just recovered (with or without analyzing) and water purified from microplastic may be obtained.

Hereinafter, examples of the present disclosure will be further described with reference to specific experimental examples. The examples and comparative examples included in the experimental examples only illustrate the present disclosure and do not limit the scope of the appended patent claims. It is obvious to those skilled in the art that various changes and modifications to the examples are possible within the scope and spirit of the present disclosure. Naturally, such changes and modifications fall within the scope of the attached patent claims.

### Experimental Example 1. Measurement of change in extent of microplastic aggregation and number of microplastic particles in supernatant depending on cycle of freezing and thawing

2 mL of a standard product containing dispersed polystyrene nanoparticles with a size of 100 to 400 nm based on the largest dimension was added to 13 mL of distilled water. The standard was then stirred at a rotation speed of 300 rpm for 5 minutes to obtain a sample of to-be-treated water containing microplastic particles. The same process was repeated to prepare three additional samples of the same to-be-treated water. As a result, a total of four to-be-treated water samples were obtained (samples 1 to 4). Thereafter, freezing the to-be-treated water samples at -20°C for 2 hours and thawing at 25°C for 2 hours was counted as 1 cycle, and then the cycle was repeated 0, 1, 5, and 10 times for samples 1 to 4, respectively. After completing the respective cycles for the samples, the precipitation of microplastic aggregates in each sample and supernatant turbidity were visually checked. The results were the same as FIG. 2.

In addition, after collecting the supernatant of each sample, nanoparticle tracking analysis (NTA) was performed by using a microscope to measure the number of microplastic particles per unit volume of the supernatant of each sample. The results were the same as FIG. 3.

Referring to FIG. 2, in the sample that did not undergo the freezing and thawing (0 times of cycle), it was confirmed that no precipitation of microplastic aggregates was observed, and the to-be-treated water sample itself was very turbid. The sample subjected to one freezing and thawing cycle showed some precipitation; turbidity of the supernatant was reduced compared to sample 1, but still some turbidity remained. The samples subjected to 5 and 10 freezing and thawing cycles, respectively, were observed to have their supernatants clear. However, more precipitation was observed in the sample introduced in 10 cycles than in the sample introduced in 5 cycles.

Referring to FIG. 3, it was confirmed that as the cycle of the freezing and thawing increased, the number of microplastic particles contained in the supernatant of the unit volume of to-be-treated water decreased. From this, it was found that as the cycle of the freezing and thawing increased, the number of aggregated microplastic particles increased, and the water quality of the supernatant improved accordingly.

### Experimental Example 2. Comparison of SEM images and image-FTIR analysis results of microplastic aggregates depending on cycle of freezing and thawing

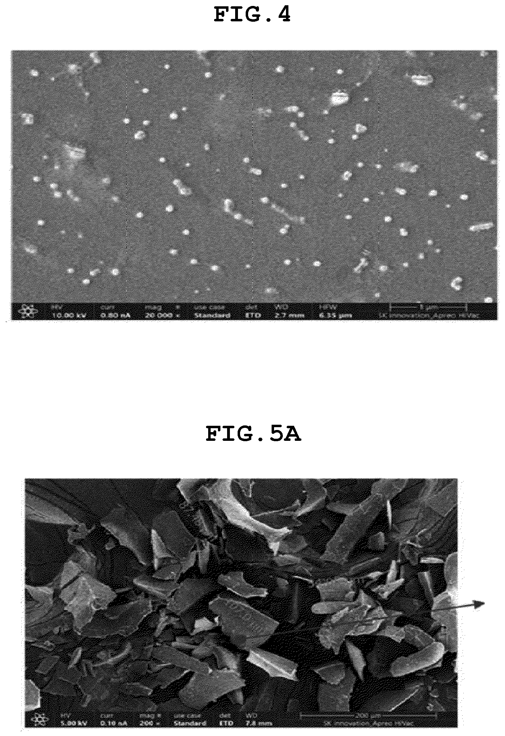

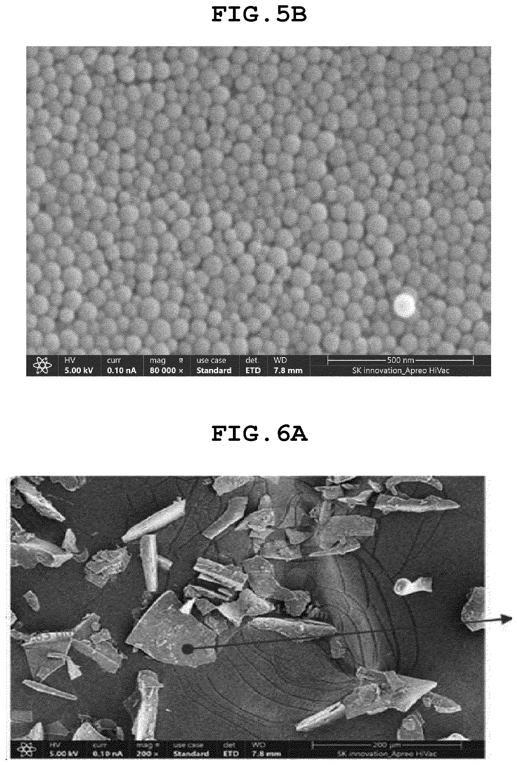

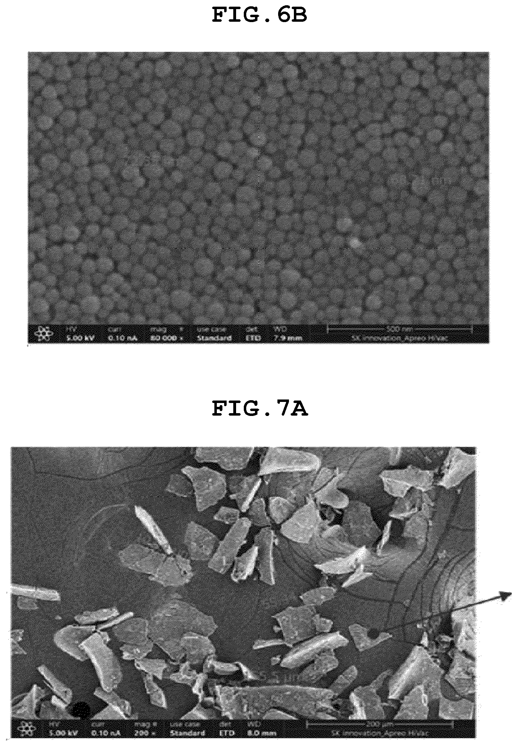

To recover microplastic aggregates by separating the supernatant of to-be-treated water, Samples 1 to 4 of Experimental Example 1 were put into a capture filter with a pore size of 25 µm. For Sample 1, no precipitation was performed, and no microplastic aggregates were recovered. For Samples 2 to 4, aggregates were recovered. The to-be-treated water of Sample 1 and the recovered microplastic aggregates of Samples 2 to 4 were observed using a scanning electron microscope (SEM). The results were the same as FIGs. 4, 5A, 6A, and 7A, respectively. In addition, in the SEM images of Samples 2 to 4 of FIGs. 5A, 6A, and 7A, when the area where the arrow starts was observed with the SEM at higher magnification, the results were the same as FIGs. 5B, 6B, and 7B, respectively.

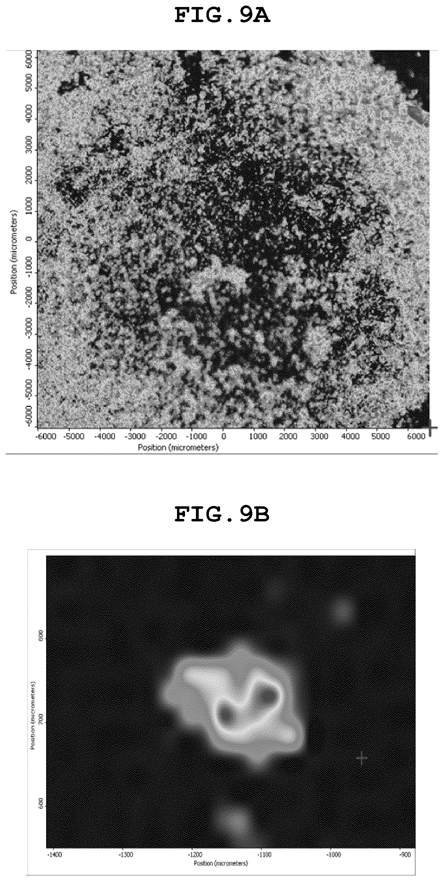

FTIR spectra were obtained for the to-be-treated water of Sample 1 and the microplastic aggregates recovered from Samples 2 to 4. Afterward, the FTIR spectra were imaged to obtain FTIR images. FIG. 8 is an FTIR image of the to-be-treated water of Sample 1. FIGs. 9A, 10A, and 11A are FTIR images of the recovered microplastic aggregatess of Samples 2 to 4. FIGs. 9B, 10B, and 11B are magnified images showing specific portions of the FTIR images of FIGS. 9A, 10A, and 11A. The results of analyzing the size of microplastic aggregates recovered from each of Samples 1 to 4 by using SEM and FTIR images are shown in Table 1.

**[Table 1]**

| Microplastic aggregate size analysis results for Samples 1 to 4 | | |
|---|---|---|
| Sample | Microplastic aggregate size (If no aggregates are formed, the size of the microplastic particles) (nm) | i-FTIR analysis result |
| Sample 1 | 148-367 | Not analyzed |
| Sample 2 | 54,000-107,000 | PS detected |
| Sample 3 | 60,000-119,000 | PS detected |
| Sample 4 | 55,000-155,000 | PS detected |

From Table 1, it was confirmed that as the cycle of the freezing and thawing increased, the size of microplastic aggregates tended to increase.

Additionally, in Sample 1, which did not undergo the cycle of the freezing and thawing, the i-FTIR spectrum corresponding to polystyrene (PS) was not detected. On the other hand, in Samples 2 to 4 which were subj ected to 1, 5, and 10 cycles of the freezing and thawing, respectively, i-FTIR spectra corresponding to polystyrene (PS) were detected.

The present disclosure has been described in detail above through specific examples. The examples are for specifically explaining the present disclosure, and the present disclosure is not limited thereto. It will be clear that modifications and improvements may be made by those skilled in the art within the technical spirit of the present disclosure.

All simple modifications or changes to the present disclosure fall within the scope of the present disclosure, and the specific scope of protection of the present disclosure will be made clear by the appended claims.

## Claims

1. A method of analyzing microplastic particles in a water system, the method comprising:
providing to-be-treated water containing microplastic particles;
freezing and thawing the to-be-treated water at least once to generate microplastic aggregates in the to-be-treated water;
recovering the microplastic aggregates; and
analyzing the recovered microplastic aggregates, optionally with respect to size, number, and/or type of the microplastic aggregates.

2. The method of claim 1, further comprising removing an impurity on the surface of the microplastic particles in the to-be-treated water before the freezing and thawing.

3. The method of claim 2, wherein the removal of the impurity on the surface of the microplastic particles is performed by any one of a strong acid or strong alkali treatment, an ultrasonic treatment, a plasma treatment, an electrochemical oxidation treatment, and an enzyme treatment, or a combination thereof.

4. The method of anyone of claims 1 to 3, further comprising filtering the to-be-treated water before the freezing and thawing.

5. The method of claim 4, wherein a filter used in the filtering has a pore size of less than 25 µm.

6. The method of any one of claims 1 to 5, wherein the freezing and thawing of the to-be-treated water is repeatedly performed until the number of microplastic particles in the supernatant of the to-be-treated water becomes 1 × 10¹⁰ pieces/mL or less.

7. The method of any one of claims 1 to 6, wherein the freezing and thawing the to-be-treated water is repeated at least twice, or 1 to 20 times, 1 to 15 times, or 5 to 10 times.

8. The method of any one of claims 1 to 7, wherein the freezing of the to-be-treated water is performed at a temperature of 0°C or lower, and the thawing of the to-be-treated water is performed at room temperature.

9. The method of any one of claims 1 to 8, wherein the recovering of the microplastic aggregates is performed using a capture filter with a pore size of 10 to 50 µm.

10. The method of any one of claims 1 to 9, wherein the analyzing of the recovered microplastic aggregates is performed by any one of Fourier transform IR spectroscopy (FTIR), Image-FTIR, Raman spectroscopy, and pyrolysis gas chromatography/mass spectrometry, or a combination thereof.

11. The method of claim 10, wherein the analyzing of the recovered microplastic aggregates is performed by image-FTIR.

12. A method of preparing water purified from microplastic, and/or of recovering microplastic from a water system, wherein the to-be-treated water contains microplastic particles, the method comprising:
providing to-be-treated water containing microplastic particles;
freezing and thawing the to-be-treated water at least once to generate microplastic aggregates in the to-be-treated water;
and
recovering the microplastic aggregates and obtaining water purified from the microplastic.

13. The method according to claim 12, which is carried out without the use of an additional coagulant, whereby the recovered microplastic aggregates and/or the purified water are free of coagulants.

14. The method of any one of claims 1 to 13, wherein the microplastic particles contained in the to-be-treated water has a particle size in a range of 10 nm to 10 cm, or in a range of 50 nm to 1000 nm, or in a range of 100 to 400 nm, respectively based on the largest dimension.
